# EUROPEAN PATENT APPLICATION

(11) **EP 4 406 518 A1**
(43) Date of publication of application: **31.07.2024**
(21) Application number: 23153678.0
(22) Date of filing: 27.01.2023
(51) Int. Cl.: A61F 5/01, A61F 5/02, A61F 5/058

(54) **BRACE FOR MANAGEMENT OF AN ANATOMICAL STRUCTURE**

(71) Applicant: Exo360 ApS, 1620 København V (DK)
(72) Inventor: TERNDRUP, Mads, 2800 Kongens Lyngby (DK); ROSENFELDT, Michael, 2800 Kongens Lyngby (DK)
(74) Representative: COPA Copenhagen Patents

(57) **Abstract**

A brace for management of an anatomical structure, the brace comprising a first brace part being adapted to be positioned to cover a first part of the anatomical structure extending over the joint and/or injury, the first brace part comprises:
a distal shell,
a compressible structure having a proximal side and a distal side and comprising a first region, the compressible structure being elastically and non-permanently deformable in a height direction normal to the proximal side of the distal shell, and the compressible structure being arranged proximal to the distal shell,
a plurality of elongated members including a first elongated member,
wherein the first elongated member extends between a first primary point and a first secondary point in the height direction,
wherein the first brace part comprises a locking mechanism, wherein the locking mechanism, in a locked state, prevents movement between the first secondary point of the first elongated member and the distal shell.

## Description

The present disclosure relates generally to orthoses, e.g., externally applied devices moving and/or stabilizing parts of an anatomical structure, such as a limb, across one or more anatomical joint(s) and/or an injury, such as to influence the structural and/or functional characteristics of the neuromuscular and skeletal system. For example, such as to allow immobilization or stabilization of an anatomical structure and pain relief and/or management thereof.

### BACKGROUND

Anatomical structures include soft tissue, joints and bones, wherein joints connect bones and allow movement in the skeletal system. Injury to anatomical structures might involve bony structures, ligaments, muscle tears, tendon ruptures and more. Most injuries are stable and will heal without or with little compromise to the functional whole across the joint. However, certain injuries are severe and cause instability across the anatomical joint, requiring internal or external fixation (either through surgical stabilization or an external cast/brace) and/or reduction (e.g., in case of dislocation), and may require realignment and/or rehabilitation to restore optimal range of motion across the affected joint.

Conventionally, a plaster cast may be provided to immobilize and/or stabilize the injured limb. However, applying a plaster cast is a time-consuming procedure and requires different tools as well as multiple healthcare providers. Furthermore, injured limbs may need multiple attempts of casting, which with a conventional plaster cast means starting over and hoping for a better second result.

Even more, when applying a plaster cast, the anatomical structure might be swollen (e.g., due to the injury). Hence, if applying a plaster cast, the cast may loosen due to reduction in swelling.

The treatment of an injury, in particular a fracture, requires fixation of the injured anatomical structure for the anatomical structure to heal correctly. If the anatomical structure is not sufficiently fixated, e.g., the fixation loosens over time, the anatomical structure may move, and the anatomical structure, such as bones and tendons, may shift to an undesirable position for healing.

### SUMMARY

It is an object of the present disclosure to provide improvements of the prior art and/or to solve or reduce problems known from the prior art, such as, for example, the problems mentioned above. More particularly, it is an object of the present disclosure to provide a solution for management of an anatomical structure, which is easy to use, allows quick and precise fitting and/or enhances flexibility and adjustability. Effectively, the disclosed solution may reduce time spent on treatment of injured limbs as well as save costs, freeing up valuable healthcare resources. Furthermore, the disclosed solution may improve the treatment outcome of injured anatomical structures.

Accordingly, a brace and a method for manufacturing the brace are disclosed. The brace is for management of an anatomical structure comprising a primary anatomical structure, a secondary anatomical structure, and a joint and/or an injury between the primary anatomical structure and the secondary anatomical structure.

The brace comprises a first brace part extending from a first primary brace part end to a first secondary brace part end and being adapted to be positioned to cover a first part of the anatomical structure extending over the joint and/or injury such that the first primary brace part end is located on the primary anatomical structure and the first secondary brace part end is located on the secondary anatomical structure.

The first brace part comprises a distal shell comprising a rigid material and having a proximal side and a distal side. The first brace part comprises a compressible structure having a proximal side and a distal side and comprising a first region. The compressible structure is elastically and non-permanently deformable in a height direction normal to the proximal side of the distal shell. The compressible structure is arranged proximal to the distal shell.

The first brace part comprises a plurality of elongated members including a first elongated member. The first elongated member extends between a first primary point and a first secondary point in the height direction. The first primary point is arranged at and travels along with the proximal side of the compressible structure within the first region of the compressible structure. The first secondary point of the first elongated member is in a primary position relative to the distal shell when the first region of the compressible structure is in a non-compressed state. The first secondary point is in a secondary position relative to the distal shell when the first region of the compressible structure is in a compressed state.

The first brace part comprises a locking mechanism. The locking mechanism, in a locked state, prevents movement between the first secondary point of the first elongated member and the distal shell.

As mentioned, a method for manufacturing a brace for management of the anatomical structure, such as the brace as disclosed above, is also disclosed. The method comprises providing a distal shell for a first brace part. The distal shell comprises a rigid material and has a proximal side and a distal side.

The method comprises providing a compressible structure and a plurality of elongated members. The compressible structure has a proximal side and a distal side and comprises a first region. The compressible structure is elastically and non-permanently deformable. The plurality of elongated members includes a first elongated member. The first elongated member extends between a first primary point and a first secondary point in the height direction.

The method comprises arranging the compressible structure and the plurality of elongated members. The compressible structure and the plurality of elongated members are arranged such that the compressible structure is arranged proximal to the distal shell, and the compressible structure being elastically and non-permanently deformable in a height direction normal to the proximal side of the distal shell.

The compressible structure and the plurality of elongated members are arranged such that the first primary point of the first elongated member is arranged at, and travels along with, the proximal side of the compressible structure within the first region of the compressible structure.

The compressible structure and the plurality of elongated members are arranged such that the first secondary point of the first elongated member is in a primary position relative to the distal shell when the first region of the compressible structure is in a non-compressed state.

The compressible structure and the plurality of elongated members are arranged such that the first secondary point of the first elongated member is in a secondary position relative to the distal shell when first region of the compressible structure is in a compressed state.

The method comprises providing a locking mechanism for the first brace part, wherein the locking mechanism, in a locked state, prevents movement between the first secondary point of the first elongated member and the distal shell.

The present disclosure provides a solution, which is quick and easy to use, is precise and adjustable, and which can be applied without any extra tools. Furthermore, the present disclosure provides for faster and more standardized manufacturing of a brace for managing an (injured) anatomical structure. It is a further advantage of the present disclosure that a brace may be provided, which reduces patient discomfort while recovering from an injury. The present disclosure further reduces the need for more complex procedures, such as surgical interventions, caused by inefficient immobilization or stabilizing of an injured anatomical structure.

An advantage of the present invention is that the compressible structure may be locked at a certain height, thereby maintaining a stable brace supporting the anatomical structure. The locking mechanism may be unlocked to readjust and refit the compressible structure to the anatomical structure, i.e., if swelling increases or decreases. In addition, the solution of the present invention is simple to use; refitting or readjusting can be performed frequently, e.g., several times a day, e.g. by the patients themselves.

The distal shell may have a thickness between the distal side and the proximal side. The distal shell may comprise a plurality of openings, including a first opening. The first elongated member may be configured to extend through the first opening. The first elongated member may be configured to extend at least partly through the compressible structure. The first elongated member may have a length longer than the thickness of the distal shell. The first elongated member may be configured to extend through the first opening such at least a part of the first elongated member protrudes from the distal side of the distal shell. The distal shell may be made of a polymer, such as an elastomer, such as polyurethane or polypropylene.

The compressible structure may be an elastomeric and/or polymeric structure. For example, the compressible structure may be made of polyurethane, such as a polyurethane elastomer. The compressible structure may comprise a least one lattice structure. The at least one lattice structure may comprise a plurality of interconnected flexible struts, which may form repeating unit cells. Such lattice structures are described in patent application EP22156131.9. The unit cells may be triangular, quadratic, pentagonal, hexagonal, or other geometric shapes. The lattice structures may differ by their geometrical shape, their thickness of struts, and/or their length of struts. The thickness (e.g. diameter) of struts may be between 0.1 mm and 5.5 mm. The length of struts may be between 0.5 mm and 10 mm, such as between 1 mm and 5 mm, such as between 1 mm and 2 mm. Geometrical shape, thickness of struts, and/or length of struts may vary along the height direction.

Alternatively, the compressible structure may be a foam and/or the compressible structure may comprise elastic elements, such as springs.

The compressible structure provides a secure immobilization of the anatomical structure in a close contact casting.

The compressible structure may be arranged such that the proximal side of the distal shell faces and/or abuts the proximal side of the compressible structure. The compressible structure may be attached to a proximal side of the distal shell. The compressible structure may have a first height in the height direction normal to the proximal side of the distal shell in a non-compressed state. The first height may be between 1.5 mm and 50 mm, such as between 5 mm and 30 mm, such as between 10 and 20 mm. The compressible structure may be adapted to: in response to increasing compression from the non-compressed state to a first compression amount in the height direction, exhibit stress increasing at a first rate, and in response to increasing compression from the first compression amount to a second compression amount in the height direction, exhibit stress being uniform or increasing at a second rate lower than the first rate.

The first compression amount may correspond to 5% of the first height, such as less than 5% of the first height. The second compression amount may correspond to more than 50% of the first height, such as 50%. 60%, 70%, 80% or 90% of the first height, or more than 60%, 70%, 80% or 90% of the first height. The second rate of stress increase between the first compression amount and the second compression amount may be less than 50%, such as less than 25%, such as less than 10%, such as less than 5%, such as less than 1% of the first rate of stress increase.

The compressible structure may further be adapted to, in response to decreasing compression from the first compression amount and/or the second compression amount to the non-compressed state, return to the first height, e.g. within a predetermined time period. The predetermined time period may be less than 30 minutes, such as less than 10 minutes, such as less than 1 minute, such as less than 30 seconds, such as less than 10 seconds, such as less than 1 second, such as less than 0.5 seconds.

The first compression amount may correspond to a stress, such as applied pressure, between 5-40 mmHg, such as between 7-36 mmHg, such as between 10-20 mmHg. The second compression amount may correspond to a pressure between 5-40 mmHg, such as between 7-36 mmHg, such as between 10-20 mmHg.

The compression amount in the non-compressed state may correspond to a stress, such as applied pressure, between 5-40 mmHg, such as between 20-36 mmHg.

The first compression amount and/or the second compression amount corresponds to a deformation which is reversible and non-permanent. Thus, in response to decreasing compression from the second compression amount, the compressible structure returns to their initial heights, i.e., the first height. The compressible structure may be configured to be reversibly compressible between the non-compressed state and the compressed state a plurality of times, such as at least 10 times.

The plurality of elongated members may include a second elongated member. The second elongated member may extend between a second primary point and a second secondary point in the height direction. The second primary point may be arranged at and may travel along with the proximal side of the compressible structure within a second region of the compressible structure. The second secondary point of the second elongated member may be in a primary position relative to the distal shell when the second region of the compressible structure is in a non-compressed state. The second secondary point may be in a secondary position relative to the distal shell when the second region of the compressible structure is in a compressed state. The locking mechanism, in the locked state, may prevent movement between the second secondary point of the second elongated member and the distal shell.

The plurality of elongated members may be made of a polymer or an elastomer, such as polyurethane, such as a polyurethane elastomer. The plurality of elongated members may be elastic. The plurality of elongated members may be configured to be jammed, e.g., between two shells.

Providing the compressible structure may comprise manufacturing the compressible structure using additive manufacturing, such as 3D printing. The compressible structure may be formed by additive manufacturing, such as 3D printing and/or resin printing. For example, the compressible structure may be formed by selective laser sintering and/or by digital light synthesis (DLS) of Carbon Inc. An exemplary process for forming the compressible structure may be found in US 2018/0264718 A1. Accordingly, the compressible structure may be a 3D printed structure, e.g. in accordance with the lattices of US 2018/0264718 A1. Alternatively, the compressible structure may be formed with thermoplastic polyurethane (TPU) using the HP Multi Jet Fusion (MJF) 3D printing technology.

The compressible structure and the plurality of elongated members may be integrally formed, e.g., by being 3D printed in a common process. Providing the compressible structure and the plurality of elongated members may comprise manufacturing the compressible structure and the plurality of elongated members in a simultaneous additive manufacturing process, such as a 3D printing process. The plurality of elongated members and the compressible structure may be made of the same material. Thereby, assembly time of the brace may be reduced and a faster and cheaper way of manufacturing a brace may be provided.

The locking mechanism may comprise an outer shell comprising a proximal side and a distal side and a plurality of openings. The method may comprise providing the outer shell. The outer shell may be arranged such that the proximal side of the outer shell faces the distal side of the distal shell. The outer shell may be arranged such that the plurality of openings of the outer shell and a plurality of openings in the distal shell are aligned. Accordingly, the plurality of openings of the outer shell and the plurality of openings in the distal shell may be aligned. The outer shell may be arranged such that the plurality of elongated members extends through the plurality of aligned openings. The plurality of elongated members may be configured to extend through the plurality of aligned openings. The outer shell may be displaceable relative to the distal shell, e.g. from a disengaged position to an engaged position, such as to transition the locking mechanism to the locked state. For example, the outer shell may be displaceable relative to the distal shell in a direction substantially perpendicular to the height direction, such as to modify the alignment of the openings. The outer shell may have a thickness between the distal side and the proximal side.

The locking mechanism, in an unlocked state, may allow movement between the secondary point of the first elongated member and the distal shell. The locking mechanism, in the unlocked state, may allow movement between the second secondary point of the second elongated member and the distal shell.

The locking mechanism may further comprise a fixation member configured to fixate the outer shell in the engaged or disengaged position. The method may comprise providing the fixation member. The fixation member may be a pin or a live hinge, e.g., a hinge between the outer shell and the distal shell. The fixation member may be flush with the outer shell in the engaged position.

The outer shell may be biased towards the engaged or disengaged position. For example, the locking mechanism may comprise an elastic member configured to bias the outer shell towards the disengaged or engaged position. The elastic member may be a live hinge, e.g., a hinge between the outer shell and the distal shell. The elastic member, the compressible structure and/or the plurality of elongated members may be integrally formed, e.g., 3D printed in a common process.

By biasing the outer shell towards the engaged or disengaged position, and thereby the locking mechanism towards the locked or unlocked position, respectively, the locking mechanism may automatically attain the locked or unlocked position thereby helping in application and/or refitting of the brace. Thus, less time and force is needed to position the outer shell and the outer shell is less likely to be positioned between the locked or unlocked position.

The locking mechanism may comprise a spacer arranged between the outer shell and the distal shell, e.g. such as to provide the brace with a desired height. The distance from the proximal side of the distal shell to the distal side of the outer shell may correspond to the height of the compressible structure in the non-compressed state. The spacer may have a height corresponding to the height of the compressible structure minus the thickness of the outer shell and minus the thickness of the distal shell, e.g. such as to achieve the corresponding height between the compressible structure and the distal shell, outer shell and spacer. The method may comprise providing a spacer and arranging the spacer between the outer shell and the distal shell.

The brace may comprise a proximal layer. The compressible structure may be arranged between the distal shell and the proximal layer. The proximal layer may be attached to the proximal side of the compressible structure, e.g., using adhesive, welding, hooks and loops, or by another attachment method known in the art. The proximal layer may be made of a hypoallergenic and/or natural material. The proximal layer may be of a breathable material and/or a material suitable to contact the skin for a longer period of time.

The method may comprise providing the proximal layer. Arranging the compressible structure may comprise arranging the compressible structure between the distal shell and the proximal layer. The method may further comprise attaching the proximal layer to the proximal side of the compressible structure, e.g., using adhesive, welding, hooks and loops, or by another attachment method known in the art.

The brace may comprise a second brace part extending from a second primary brace part end to a second secondary brace part end. The second brace part may be adapted to be positioned to cover a second part of the anatomical structure. The second part of the anatomical structure may be opposite the first part of the anatomical structure. The second brace part may be adapted to be positioned to cover the second part of the anatomical structure extending over the joint and/or injury such that the second primary brace part end is located on the primary anatomical structure and the second secondary brace part end is located on the secondary anatomical structure. Accordingly, the method may comprise providing such second brace part. The second brace part may comprise a second distal shell. The second brace part may be substantially similar to the first brace part but shaped and/or configured to cover the second part of the anatomical structure. The second brace part may comprise the same features as described for the first brace part. However, in some examples, the second brace part comprise only some of the features of the first brace part and/or in some examples, the second brace part comprises some features not part of the first brace part.

The first brace part may be attached to the second brace part. The second brace part may comprise a second distal shell comprising a rigid material and having a proximal side and a distal side. In some examples, the second brace part may be without a compressible structure, i.e., providing a hard shell to be positioned to cover the second part of the anatomical structure. Alternatively, the second brace part may comprise a second compressible structure having a proximal side and a distal side and comprising a first region. The second compressible structure may be elastically and non-permanently deformable in a second height direction normal to the proximal side of the second distal shell. The second compressible structure may be arranged proximal to the second distal shell.

The second brace part may comprise a plurality of elongated members including a first elongated member. The first elongated member of the second brace part extends between a first primary point and a first secondary point in the second height direction. The first primary point is arranged at and travels along with the proximal side of the second compressible structure within the first region of the second compressible structure. The first secondary point of the first elongated member of the second brace part is in a primary position relative to the second distal shell when the first region of the second compressible structure is in a non-compressed state. The first secondary point is in a secondary position relative to the second distal shell when the first region of the second compressible structure is in a compressed state.

The second brace part may comprise a second locking mechanism. The second locking mechanism, in a locked state, prevents movement between the first secondary point of the first elongated member of the second brace part and the second distal shell.

The brace may comprise one or more fasteners adapted to fasten the brace to the anatomical structure. The one or more fasteners may include one or more straps. The fasteners, such as the straps, may include hook and loop fasteners. The fasteners, such as the straps, may facilitate incrementally tightening or loosening the fastening of the brace to the anatomical structure. The fasteners, such as the straps, may allow for easy opening and re-closure, such as to allow repositioning of the brace. The one or more fasteners, such as the straps, may be adapted to fasten the first brace part to the second brace part, e.g. so as to fasten the brace around the anatomical structure. The method may comprise providing fasteners and attaching the first brace part to the second brace part using the fasteners.

The second compressible structure may have a second height in the second height direction normal to the second proximal side of the second distal shell in a non-compressed state. The second height may be between 1.5 mm and 50 mm, such as between 5 and 30 mm, such as between 10 and 20 mm. Furthermore, like for the compressible structure of the first brace part, the second compressible structure may be adapted to: in response to increasing compression from the non-compressed state to a primary compression amount in the height direction, exhibit stress increasing at a primary rate, and in response to increasing compression from the primary compression amount to a secondary compression amount in the height direction, exhibit stress being uniform or increasing at a secondary rate lower than the primary rate.

The primary compression amount may correspond to 5% of the second height, such as less than 5% of the second height. The secondary compression amount may correspond to more than 50% of the second height, such as 50%. 60%, 70%, 80% or 90% of the second height, or more than 60%, 70%, 80% or 90% of the second height. The secondary rate of stress increase between the primary compression amount and the secondary compression amount may be less than 50%, such as less than 25%, such as less than 10%, such as less than 5%, such as less than 1% of the primary rate of stress increase.

The second compressible structure may further be adapted to, in response to decreasing compression from the primary compression amount and/or the secondary compression amount to the non-compressed state, return to the second height, e.g., within a predetermined time period, which may be similar to the predetermined time period as described in relation to the compressible structure of the first brace part.

The primary compression amount and/or the secondary compression amount correspond to a deformation which is reversible and non-permanent. Thus, in response to decreasing compression from the primary compression amount and/or secondary compression amount, the one or more lattice structures return to their initial heights, i.e. the second height.

The primary compression amount may correspond to a stress, such as applied pressure, between 5-40 mmHg, such as between 7-36 mmHg, such as between 10-20 mmHg. The secondary compression amount may correspond to a pressure between 5-40 mmHg, such as between 7-36 mmHg, such as between 10-20 mmHg.

The distal shell of the first brace part and/or the second distal shell of the second brace part may be formed to fit the anatomical structure. The proximal side of distal shell of the first brace part and/or the second distal shell of the second brace part may be concave, such as to at least partially enclose the respective part of the anatomical structure, i.e., the first part or the second part of the anatomical structure

The method for manufacturing the brace may comprise obtaining a three-dimensional model of the anatomical structure, e.g. including the location of the joint and/or the injury. Forming the one compressible structure may be based on the three-dimensional model. The three-dimensional model may include information of tissue type at a plurality of locations of the anatomical structure, such as positions of bony prominences, soft tissue, ligaments, superficial nerves etc.

In the present disclosure, the terms "distal" and "proximal", such as a distal/proximal side, refer to arrangements further from or closer to the center of anatomical structure, respectively.

### BRIEF DESCRIPTION OF THE FIGURES

Embodiments of the disclosure will be described in more detail in the following with regard to the accompanying figures. The figures show one way of implementing the present disclosure and are not to be construed as being limiting to other possible embodiments falling within the scope of the attached claim set.
Fig. 1 is a schematic diagram illustrating an exemplary brace,
Fig. 2 is a schematic diagram illustrating an exemplary anatomical structure,
Fig. 3a-3c are schematic diagrams illustrating the exemplary brace,
Fig. 4 is a schematic diagram illustrating a cross sectional view of the first brace part or the second brace part of the exemplary brace,
Figs. 5a-5b schematically illustrates stress-strain relationships,
Figs. 6ai-6dii are schematic diagrams illustrating the exemplary brace, and
Figs. 7a-7b are schematic diagrams illustrating exemplary braces.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment need not have all the aspects or advantages shown. An aspect or an advantage described in conjunction with a particular embodiment is not necessarily limited to that embodiment and can be practiced in any other embodiment even if not so illustrated, or if not so explicitly described.

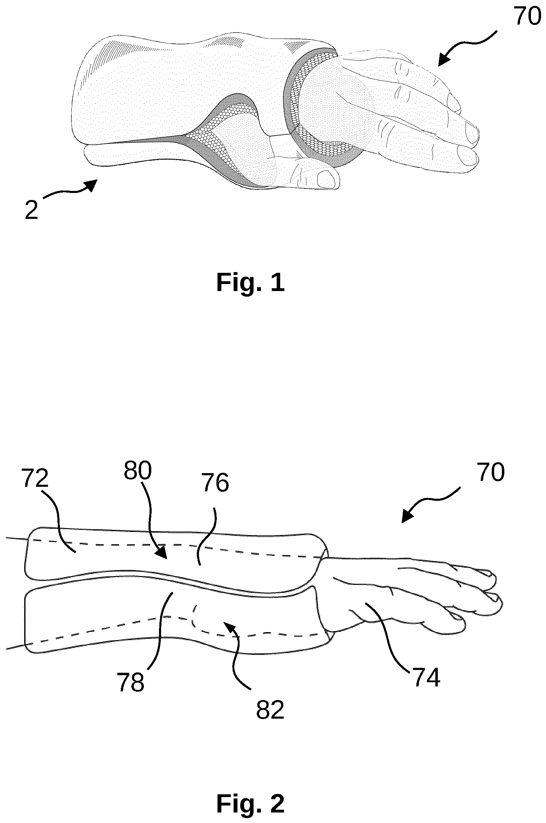

Fig. 1 is a schematic diagram illustrating an exemplary brace 2 according to the present disclosure. The brace 2 is attached to an anatomical structure 70, in the present case a wrist. The exemplary anatomical structure 70 is further schematically illustrated in Fig. 2. Although in the presently illustrated example, the anatomical structure 70 is a wrist, it should be understood that the brace according to the present disclosure may be shaped to be fitted to other anatomical structures, such as an ankle, a knee, an elbow etc.

The anatomical structure 70 comprises a primary anatomical structure 72, e.g., the forearm, and a secondary anatomical structure 74, e.g., the hand. The anatomical structure 70 further comprises a joint 76, e.g., the wrist and/or an injury 78, e.g., a bone fracture, tissue damage, or similar. The joint 76 and/or the injury 78 is located between the primary anatomical structure 72 and the secondary anatomical structure 74.

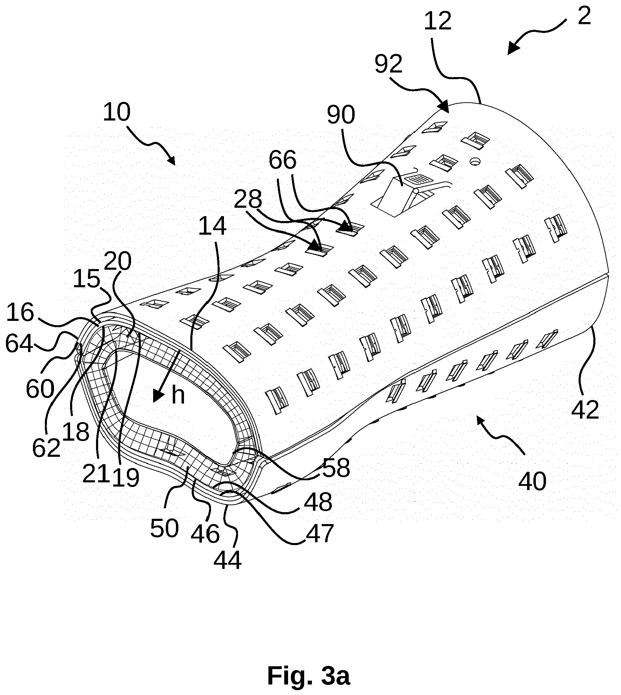

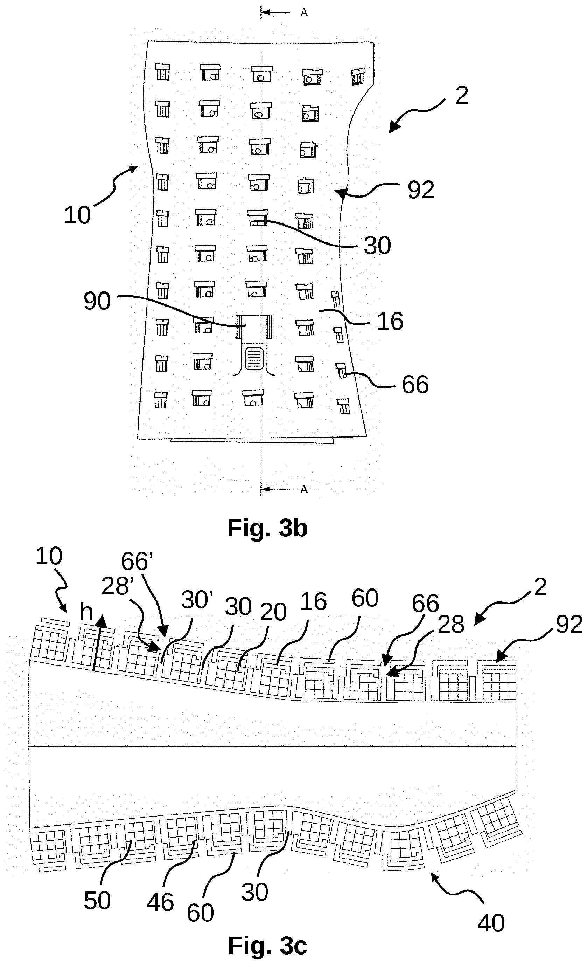

Fig. 3a-3c are schematic diagrams illustrating the exemplary brace 2. Fig. 3a is an isometric view of an exemplary brace 2. Fig 3b is a top or bottom view of an exemplary brace 2. Fig. 3c is a cross sectional view of an exemplary brace 2 seen from the line A of Fig. 3b.

The brace 2 comprises a first brace part 10 extending from a first primary brace part end 12 to a first secondary brace part end 14. The first brace part 10 is adapted to be positioned to cover a first part 80 (see Fig. 2) of the anatomical structure 70 extending over the joint 76 and/or injury 78, such that the first primary brace part end 12 is located on the primary anatomical structure 72 and the first secondary brace part end 14 is located on the secondary anatomical structure 74.

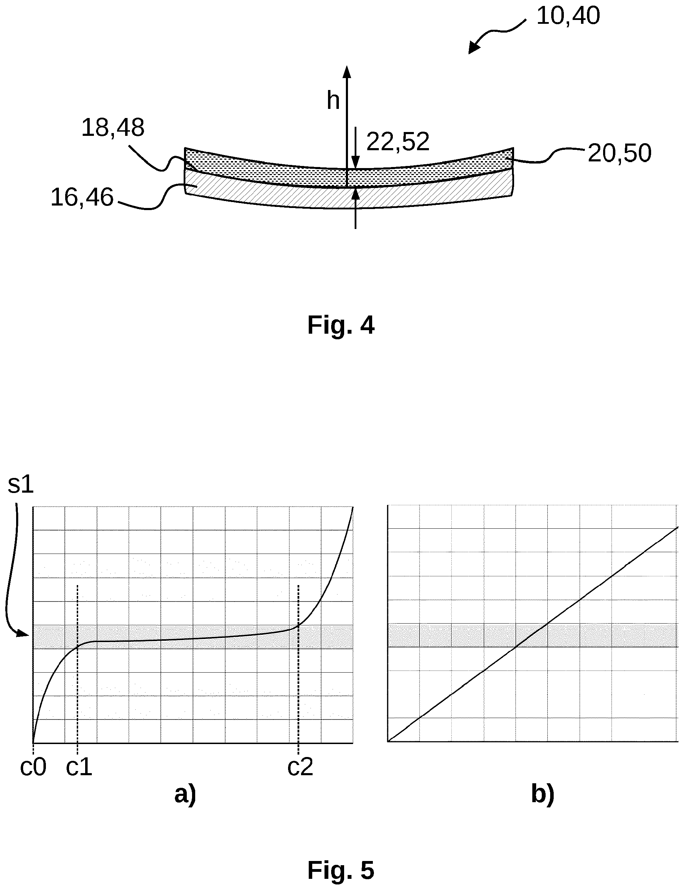

The first brace part 10 comprises a distal shell 16. The distal shell 16 is sufficiently rigid to provide the necessary stability of the brace 2 to support the anatomical structure 70. The distal shell 16 comprises a proximal side 18 and a distal side 15. The proximal side 18 is concave to at least partially enclose the first part 80 of the anatomical structure 70. The first brace part 10 further comprises a compressible structure 20 comprising a distal side 19 and a proximal side 21. The proximal side 21 of the compressible structure 20 is attached to the proximal side 18 of the distal shell 16. The compressible structure 20 may be elastomeric and/or polymeric and may comprise lattice structures. Although not specifically illustrated, it is noted that the lattice structures, such as their geometrical shape, thickness of struts, and/or length of struts, may vary along the height of the lattice structure, i.e., along the height direction h (cf. Fig. 4).

Alternatively, the compressible structure 20 may be a foam or comprise other elastic materials. In some examples, the compressible structure 20 may be divided into a plurality of individually manufactured portions each being attached to the proximal side 18. In other examples, the compressible structure 20 may be formed as a single structure extending from the first primary brace part end 12 to the first secondary brace part end 14. The compressible structure 20 may be attached to the proximal side 18 by gluing, by hooks and loops, or by another attachment method known in the art. The compressible structure 20 may be only lightly attached, as the positioning of the brace 2 on the anatomical structure will help maintain the position of the compressible structure 20 with respect to the distal shell 16.

The first brace part 10 may comprise a proximal layer 58. The proximal layer 58 may be formed by the compressible structure 20 or be a separate layer. The proximal layer 58 may be a material suitable for close contact with the skin for a longer period of time, such as hypoallergenic and/or sweat absorbing or resistant materials.

The brace 2, as exemplified in Figs. 3a-3c, comprises a second brace part 40. The second brace part 40 extends from a second primary brace part end 42 to a second secondary brace part end 44. The second brace part 40 is adapted to be positioned to cover a second part 82 (see Fig. 2) of the anatomical structure 70 extending over the joint 76 and/or injury 78, such that the second primary brace part end 42 is located on the primary anatomical structure 72 and the second secondary brace part end 44 is located on the secondary anatomical structure 74. The second part 82 of the anatomical structure may be opposite the first part 80 of the anatomical structure. For example, the first part 80 may be a posterior side of the forearm, wrist, hand, as illustrated in Fig. 2, and the second part 82 may be an anterior side of the forearm, wrist, hand, as illustrated in Fig. 2.

The second brace part 40 comprises a second distal shell 46. The second distal shell 46 is sufficiently rigid to provide the necessary stability of the brace 2 to support the anatomical structure 70. The second distal shell 46 comprises a second proximal side 48. The second proximal side 48 is concave to at least partially enclose the second part 82 of the anatomical structure 70. The second brace part 40 further comprises a second compressible structure 50 attached to the second proximal side 48 of the second distal shell 46. The second compressible structure 50 may be elastomeric and/or polymeric and may comprise lattice structures. In some examples, the second compressible structure 50 may be divided into a plurality of individually manufactured portions each being attached to the second proximal side 48. In other examples, the second compressible structure 50 may be formed as a single structure extending from the second primary brace part end 42 to the second secondary brace part end 44. The second compressible structure 50 may be attached to the second proximal side 48 by gluing, by hooks and loops, or by another attachment method known in the art. The second compressible structure 50 may be only lightly attached, as the positioning of the brace 2 on the anatomical structure will help maintain the position of the second compressible structure 50 with respect to the second distal shell 46. In some examples, the second brace part 40 may be omitted. In some examples, the second brace part 40 may omit the second compressible structure 50.

The brace 2 may comprise fasteners (not illustrated) adapted to fasten the brace 2 to the anatomical structure 70. For example, the fasteners may include straps, such as hook and loop straps. The fasteners may facilitate incrementally tightening or loosening of the brace. The fasteners may be adapted to fasten the first brace part 10 to the second brace part 40, so as to fasten the brace 2 around the anatomical structure 70.

In the following, features of the first brace part 10 are described. However, the second brace part 40 may comprise some or all of the same features as described for the first brace part 10.

The first brace part 10 comprises a outer shell 60. The outer shell 60 comprises a proximal side 62 and a distal side 64 and a plurality of openings 66. The outer shell 60 is arranged such that proximal side 62 of the outer shell 60 faces the distal side 15 of the distal shell 16. The outer shell 60 is arranged spaced apart from the distal shell 16, e.g. in the height direction h. The plurality of openings 66 of the outer shell 60 are aligned with the plurality of openings 28 of the distal shell 16. The outer shell 60 is displaceable relative to the distal shell 16, and in the particular embodiment of Figs. 3a- 3c, the outer shell 60 is displaceable relative to the distal shell 16 in a direction perpendicular to the height direction h, such as along the direction of the forearm, of the illustrated example.

The first brace part 10 comprises a plurality of elongated members 30. The plurality of elongated members 30 includes a first elongated member 30'. The plurality of elongated members 30 are configured to extend through the plurality of openings 28, 66. The first elongated member 30' is configured to extend through the first opening 28' of the distal shell 16 and the first opening 66' of the outer shell 60.

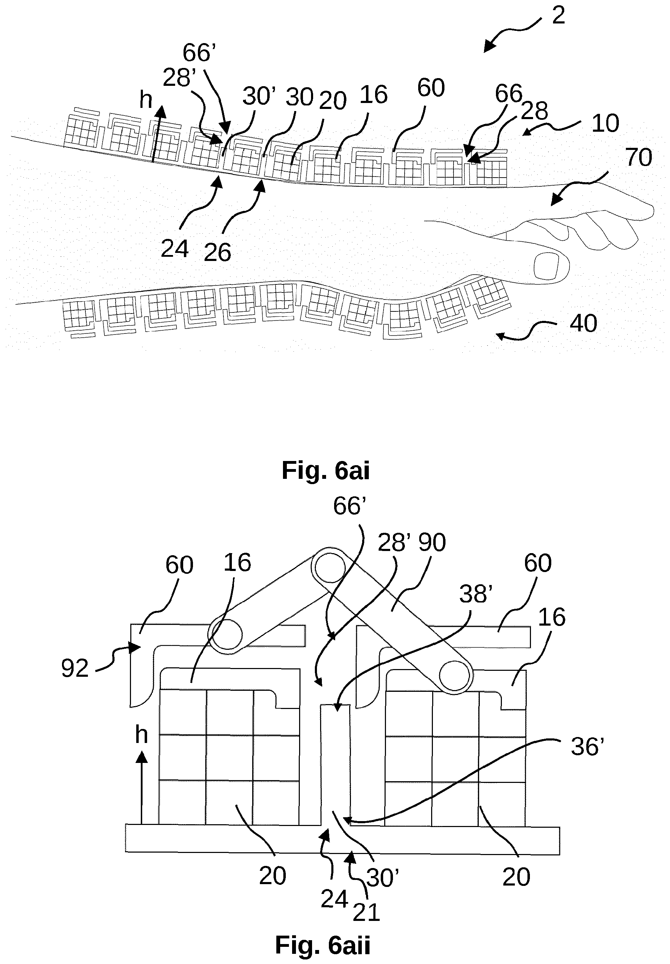

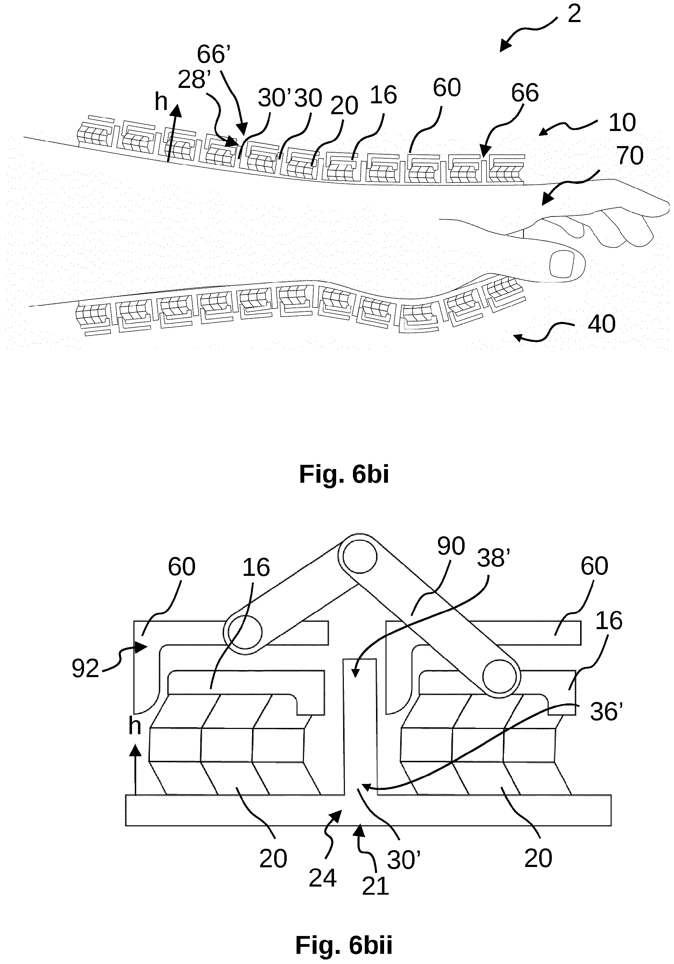

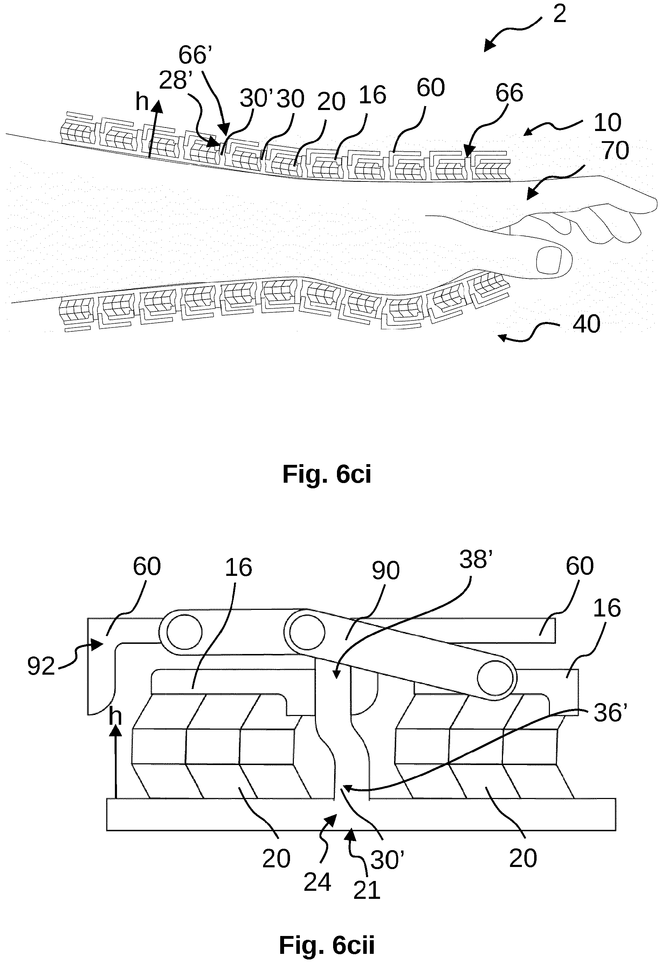

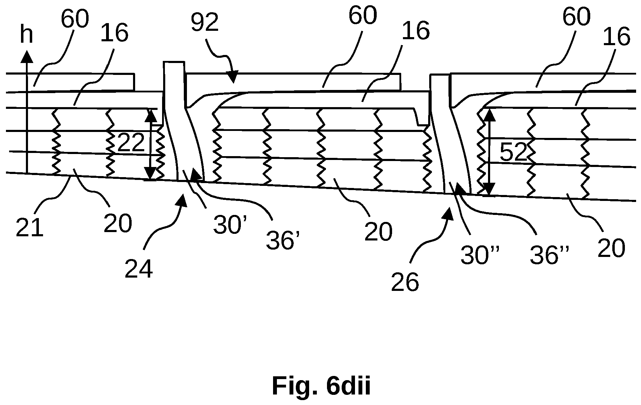

The first brace part 10 further comprises a locking mechanism 92. The locking mechanism 92 may in a locked state prevent movement of the elongated member 30 relative to the distal shell 16 (see Figs. 6ai-6dii).

Fig. 4 is a schematic diagram illustrating a cross sectional view of the first brace part 10 or the second brace part 40 of the exemplary brace 2, as illustrated in the previous figures. The compressible structures 20, 50 have respective first and second heights 22, 52 in a height direction h normal to the respective proximal side 18, 48 of the distal shell 16,46. The illustrated example shows the heights 22, 52 in a non-compressed state. In case of compression, i.e., a decrease in the height, the compressible structures 20, 50 are adapted to exhibit stress, which when being compressed from the non-compressed state to a first compression amount in the height direction h, increases at a first rate. When further being compressed from the first compression amount to a second compression amount, the compressible structures 20, 50 are adapted to exhibit stress being uniform or increasing at a second rate lower than the first rate.

The stress strain relationship is illustrated in Fig. 5a, which schematically illustrates an exemplary stress-strain curve for preferred compressible structures 20, 50, such as a lattice structure. The horizontal axis is strain, i.e., the amount of compression, whereas the vertical axis shows stress, i.e., pressure or force, exhibited by the lattice structure when being compressed. As mentioned, when compressed from the non-compressed state c0 to a first compression amount c1, the compressible structures 20, 50 exhibits stress increasing at a first rate. This may be referred to as the linear elasticity zone. When further being compressed from the first compression amount c1 to a second compression amount c2, the compressible structures 20, 50 are adapted to exhibit stress being uniform or increasing at a second rate lower than the first rate. This may be referred to as a plateau zone. In response to decreasing compression from the second compression amount c2 and/or from the first compression amount c1 to the non-compressed state c1, the compressible structures 20, 50 should preferably return to their initial height 22, 52. Returning to the initial height may take some time, but is preferably a short time, i.e., seconds or milliseconds. The first compression amount c1 may correspond to 5% compression, such as less than 5% compression. The second compression amount c2 may correspond to more than 50% compression, such as 50%. 60%, 70%, 80% or 90% compression, or more than 60%, 70%, 80% or 90% compression. Thus, the first compression amount and the second compression amount may correspond to non-permanent and reversible deformation.

When being further compressed beyond the second compression amount c2, the compressible structures 20, 50 may exhibit stress increasing at a third rate. For the purpose of the brace as disclosed herein, it is intended that the lattice structures are compressed to between the first compression amount c1 and the second compression amount c2, when being fastened to the anatomical structure. Thereby, substantially the same pressure may be applied to the anatomical structure for various amounts of compression of the compressible structures. Thereby allowing the same pressure to be applied to the anatomical structure following a change of the anatomical structure, e.g. caused by reduced or increased swelling.

Fig. 5b schematically illustrates, for comparison, an exemplary stress-strain curve for a regular padding of a conventional brace or cast.

The greyed area in the middle of the graphs in Figs. 5a and 5b, illustrates optimal pressure or force s1 to be applied to the anatomical structure. As seen, the compressible structures 20, 50 are preferably designed such that the stress exhibited in the plateau zone, i.e., between the first compression amount c1 and the second compression amount c2, is within this optimal value s1. For comparison, as seen in Fig. 5b, only a very specific compression of the padding results in a stress corresponding to the optimal value, which means that, for example, a reduced swelling will result in the brace becoming loose and not sufficiently supporting the anatomical structure as intended.

Figs. 6ai-6dii are schematic diagrams illustrating an exemplary brace 2 comprising a compressible structure 20, 50. The illustrated examples show the compressible structure 20 as also shown in Figs. 2-3. However, it should be understood that the second compressible structure 50 of Figs. 2-3 may comprise the same overall features, although possibly being differently shaped. Thus, for simplicity, the present explanation with reference to Figs. 6ai-6dii is provided with respect to the first brace part 10, but the description may also apply to the second brace part 40.

Figs. 6ai, 6bi, 6ci illustrate a cross-sectional view of the brace 2 and Figs. 6aii, 6bii, and 6cii illustrate an enlarged cross-sectional view of the brace 2.

The brace 2 is described in detail in relation to the previous figures, e.g., Figs. 3a-3d. The enlarged region illustrated in Figs. 6aii, 6bii, 6cii, 6dii illustrates the first compressible structure 20 attached to the first distal shell 16. The first outer shell 60 is arranged distally to the first distal shell 16. The plurality of elongated members 30 including the first elongated member 30' are configured to extend through the plurality of openings 28, including the first opening 28', of the first distal shell 16 and the plurality of openings 66, including the first opening 66', of the first outer shell 60. The first distal shell 16 and the first outer shell 60 are arranged such that the openings 28, 66 are aligned.

In the following the features of the first elongated member 30' is described. However, the description also applies to any other of the plurality of elongated members 30. The first elongated member 30' extends between a first primary point 36' and a first secondary point 38' in the height direction h. The first primary point 36' is arranged at and travels along with the proximal side 21 of the compressible structure 20 within the first region 24 of the first compressible structure 20. The first elongated member 30' extends through the aligned first openings 28', 66'.

Figs. 6ai-6aii illustrates the compressible structure 20 in a non-compressed state. The first secondary point 38' of the first elongated member 30' is in a primary position relative to the first distal shell 16 when the first region 24 of the compressible structure 20 is in this non-compressed state. The outer shell 60 is in a disengaged position, the locking mechanism 92 is in an unlocked state.

Figs. 6bi-6bii illustrate the compressible structure 20 in a compressed state. The first secondary point 38' is in a secondary position relative to the distal shell 16 when the first region 24 of the compressible structure 20 is in this compressed state. The outer shell 60 is in a disengaged position and the locking mechanism 92 is in an unlocked state

Figs. 6ci-6cii illustrate the compressible structure 20 in a compressed state and outer shell 60 is in an engaged position thereby positioning the locking mechanism 92 in a locked state. In the engaged position the outer shell 60 is displaced relative to the distal shell 16 in a direction perpendicular to the height direction h, such as to transition the locking mechanism 92 to a locked state. The outer shell 60 may thus act as a locking mechanism, wherein the locking mechanism, in a locked state, prevents movement between the first secondary point 38' of the first elongated member 30' and the distal shell 16, e.g., by jamming or clamping the first elongated member 30' between the distal shell 16 and the outer shell 60. Other suitable locking mechanisms that prevent movement of the first elongated member 30' may alternatively be applied. Fig. 6cii also illustrates a fixation member 90 fixating the outer shell 60 in the engaged position. The fixation member 90 prevents the outer shell 60 from further displacement. The fixation member 90 may also provide a biasing of the outer shell 60 towards a disengaged position, such that, when released, the outer shell 60 is biased towards returning to the disengaged position. The fixating member 90 is flush with the outer shell 60 when the outer shell 60 is fixated in the engaged position. The fixation member 90 may be a clip or a live hinge. However, other means of fixating the locking mechanism 92 may also be applied.

In the compressed, locked state and engaged position of Figs. 6ci-6cii the anatomical structure 70 is securely immobilized in a close contact casting of the brace 2. The brace 2 may be transitioned between the states illustrated in Figs. 6a-6c a plurality of times, such as to obtain the perfect fit, e.g. a plurality of times during a day. The first compressible structure 20 is elastic and returns towards the initial height of Fig. 6ai if the locking mechanism is unlocked. Thereby, the brace may be refitted by transitioning the locking mechanism to the unlocked state. Afterwards, the locking mechanism may be locked to ensure a tight and stable fit.

Fig. 6dii illustrates an enlarged view of the first compressible structure 20, but the same features may also apply for the second compressible structure. The first compressible structure 20 comprises a plurality of regions including a first region 24 and a second region 26. The first primary point 36' of the first elongated member 30' is arranged at and travels along with the first proximal side 21 of the first compressible structure 20 within the first region 24 of the first compressible structure 20.

The first primary point 36" of the second elongated member 30" is arranged at and travels along with the first proximal side 21 of the first compressible structure 20 within the second region 26 of the first compressible structure 20. The first region 24 and the second region 26 of the first compressible structure 20 may be compressed at different amounts as illustrated in Fig. 6dii. The locking mechanism, e.g., the first outer shell 60 may be locked, such that the relative position of the first primary point 36', 36" of elongated members 30', 30" to the first distal shell 16 is different. For example, the first height 22 of the first compressible structure 20 at the first region 24 may be smaller than the second height 52 of the first compressible structure 20 at the second region 26. In the locked state and/or engaged position, the first height 22 and the second height 52 are kept constant, e.g., the compressible structure 20 does not compress further.

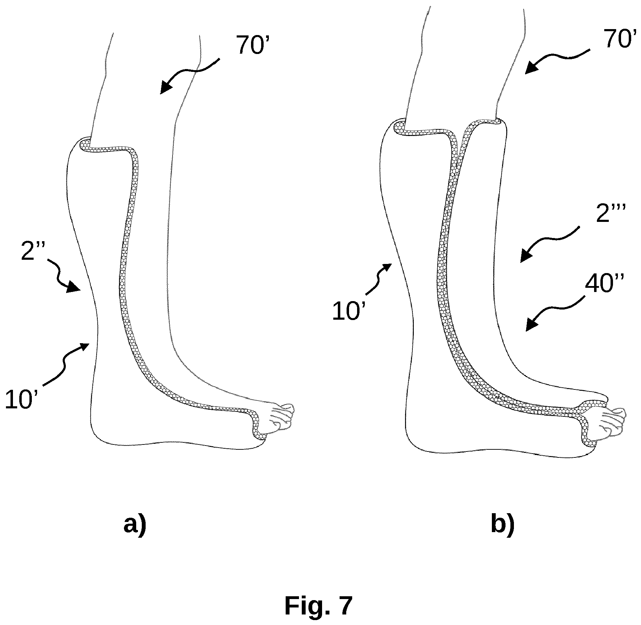

Figs. 7a-7b are schematic diagrams each illustrating an exemplary brace 2", 2‴, according to the present disclosure. More specifically Figs. 7a and 7b are examples of braces 2", 2‴ being adapted to be attached to another exemplary anatomical structure 70', wherein the anatomical structure 70' as depicted in Figs. 7a and 7b, comprises a leg, an ankle and/or a foot. Other than being shaped differently the braces 2" 2‴ are similar to the braces as described previously. Fig. 7a show an example of a brace 2" comprising only the first brace part 10'. Fig. 7b shows an example of a brace 2‴ comprising both the first brace part 10' and the second brace part 40".

The disclosure has been described with reference to a preferred embodiment. However, the scope of the invention is not limited to the illustrated embodiment, and alterations and modifications can be carried out without deviating from the scope of the invention.

Throughout the description, the use of the terms "first", "second", "third", "fourth", "primary", "secondary", "tertiary" etc. does not imply any particular order or importance but are included to identify individual elements. Furthermore, the labelling of a first element does not imply the presence of a second element and vice versa.

### ITEMS

Exemplary embodiments of the present disclosure are set out in the following items:
1. A brace for management of an anatomical structure comprising a primary anatomical structure, a secondary anatomical structure, and a joint and/or an injury between the primary anatomical structure and the secondary anatomical structure,
   the brace comprising a first brace part extending from a first primary brace part end to a first secondary brace part end and being adapted to be positioned to cover a first part of the anatomical structure extending over the joint and/or injury such that the first primary brace part end is located on the primary anatomical structure and the first secondary brace part end is located on the secondary anatomical structure,
   the first brace part comprises:
      - a distal shell comprising a rigid material and having a proximal side and a distal side,
      - a compressible structure having a proximal side and a distal side and comprising a first region, the compressible structure being elastically and non-permanently deformable in a height direction normal to the proximal side of the distal shell, and the compressible structure being arranged proximal to the distal shell,
      - a plurality of elongated members including a first elongated member,
         ∘ wherein the first elongated member extends between a first primary point and a first secondary point in the height direction,
         ∘ wherein the first primary point is arranged at and travels along with the proximal side of the compressible structure within the first region of the compressible structure,
         ∘ wherein the first secondary point of the first elongated member is in a primary position relative to the distal shell when the first region of the compressible structure is in a non-compressed state, and wherein the first secondary point is in a secondary position relative to the distal shell when the first region of the compressible structure is in a compressed state,
   wherein the first brace part comprises a locking mechanism, wherein the locking mechanism, in a locked state, prevents movement between the first secondary point of the first elongated member and the distal shell.
2. Brace according to item 1, wherein the distal shell comprises a plurality of openings, including a first opening, and wherein the first elongated member is configured to extend through the first opening.
3. Brace according to any of the preceding items, wherein the distal shell is made of a polymer, such as an elastomer, such as polyurethane or polypropylene.
4. Brace according to any of the preceding items, wherein the plurality of elongated members is made of a polymer or an elastomer, such as a polyurethane elastomer.
5. Brace according to any of the preceding items, wherein the plurality of elongated members includes a second elongated member,
   - wherein the second elongated member extends between a second primary point and a second secondary point in the height direction,
   - wherein the second primary point is arranged at and travels along with the proximal side of the compressible structure within a second region of the compressible structure,
   - wherein the second secondary point of the second elongated member is in a primary position relative to the distal shell when the second region of the compressible structure is in a non-compressed state, and wherein the second secondary point is in a secondary position relative to the distal shell when the second region of the compressible structure is in a compressed state,
   wherein the locking mechanism, in the locked state, prevents movement between the second secondary point of the second elongated member and the distal shell.
6. Brace according to any of the preceding items, wherein the compressible structure comprises at least one lattice structure.
7. Brace according to any of the preceding items, wherein the compressible structure is a foam.
8. Brace according to any of the preceding items, wherein the compressible structure comprises elastic elements, such as springs.
9. Brace according to any of the preceding items, wherein the compressible structure is a 3D printed structure.
10. Brace according to any of the preceding items, wherein the compressible structure and the plurality of elongated members are integrally formed, e.g., by being 3D printed in a common process.
11. Brace according to any of the preceding items wherein the plurality of elongated members and the compressible structure are made of the same material.
12. Brace according to any of the preceding items wherein the compressible structure is configured to be reversibly compressible between the non-compressed state and the compressed state a plurality of times, such as at least 10 times.
13. Brace according to any of the preceding items comprising a proximal layer, wherein the compressible structure is arranged between the distal shell and the proximal layer, and wherein the proximal layer is attached to the proximal side of the compressible structure.
14. Brace according to any of the preceding items, wherein the proximal layer is made of a hypoallergenic and/or natural material.
15. Brace according to any of the preceding items, wherein the locking mechanism, in an unlocked state, allows movement between the secondary point of the first elongated member and the distal shell.
16. Brace according to any of the preceding items comprising a second brace part comprising:
   - a distal shell comprising a rigid material and having a proximal side and a distal side,
   - a compressible structure having a proximal side and a distal side and comprising a first region and a second region, the compressible structure being elastically and non-permanently deformable in a height direction normal to the proximal side of the distal shell, and the compressible structure being arranged proximal to the distal shell,
   - a plurality of elongated members including a first elongated member,
      ∘ wherein the first elongated member extends between a first primary point and a first secondary point in the height direction,
      ∘ wherein the first primary point is arranged at and travels along with the proximal side of the compressible structure within the first region of the compressible structure,
      ∘ wherein the first secondary point of the first elongated member is in a primary position relative to the distal shell when the first region of the compressible structure is in a non-compressed state, and wherein the first secondary point is in a secondary position relative to the distal shell when the second region of the compressible structure is in a compressed state,
      wherein the second brace part comprises a locking mechanism, wherein the locking mechanism, in a locked state, prevents movement between the first secondary point of the first elongated member and the distal shell.
17. Brace according to any of the preceding items as dependent on item 2, wherein the locking mechanism comprises an outer shell comprising a proximal side and a distal side and a plurality of openings, wherein the outer shell is arranged such that the proximal side of the outer shell faces the distal side of the distal shell,
   wherein the plurality of openings of the outer shell and the plurality of openings in the distal shell are aligned, and wherein the plurality of elongated members is configured to extend through the plurality of aligned openings, and
   wherein the outer shell is displaceable relative to the distal shell from a disengaged position to an engaged position, such as to transition the locking mechanism to the locked state.
18. Brace according to item 17 wherein the locking mechanism further comprises a fixation member configured to fixate the outer shell in the engaged or disengaged position.
19. Brace according to any of the items 17-18 wherein the outer shell is displaceable relative to the distal shell in a direction substantially perpendicular to the height direction such as to modify the alignment of the openings.
20. Brace according to any of the preceding items wherein the outer shell is biased towards the engaged or disengaged position.
21. Method for manufacturing a brace for management of an anatomical structure comprising a primary anatomical structure, a secondary anatomical structure, and a joint and/or an injury between the primary anatomical structure and the secondary anatomical structure, the method comprising:
   - providing a distal shell for a first brace part, the distal shell comprising a rigid material and having a proximal side and a distal side,
   - providing a compressible structure and a plurality of elongated members,
      ∘ the compressible structure having a proximal side and a distal side and comprising a first region, the compressible structure being elastically and non-permanently deformable,
      ∘ the plurality of elongated members includes a first elongated member, wherein the first elongated member extends between a first primary point and a first secondary point in the height direction,
   - arranging the compressible structure and the plurality of elongated members,
      ∘ such that the compressible structure is arranged proximal to the distal shell, and the compressible structure being elastically and non-permanently deformable in a height direction normal to the proximal side of the distal shell,
      ∘ such that the first primary point of the first elongated member is arranged at, and travels along with, the proximal side of the compressible structure within the first region of the compressible structure,
      ∘ such that the first secondary point of the first elongated member is in a primary position relative to the distal shell when the first region of the compressible structure is in a non-compressed state, and
      ∘ such that the first secondary point of the first elongated member is in a secondary position relative to the distal shell when first region of the compressible structure is in a compressed state,
   - providing a locking mechanism for the first brace part, wherein the locking mechanism, in a locked state, prevents movement between the first secondary point of the first elongated member and the distal shell.
22. Method according to item 21, wherein providing the compressible structure comprises manufacturing the compressible structure using additive manufacturing, such as 3D printing.
23. Method according to any of the items 21-22, wherein providing the compressible structure and the plurality of elongated members comprises manufacturing the compressible structure and the plurality of elongated members in a simultaneous additive manufacturing process, such as a 3D printing process.
24. Method according to any of the items 21-23 comprising providing a proximal layer, and wherein arranging the compressible structure comprises arranging the compressible structure between the distal shell and the proximal layer, the method further comprising attaching the proximal layer to the proximal side of the compressible structure.
25. Method according to any of the items 21-24 comprising:
   - providing an outer shell comprising a proximal side and a distal side and a plurality of openings,
   - arranging the outer shell
      ∘ such that the proximal side of the outer shell faces the distal side of the distal shell,
      ∘ such that the plurality of openings of the outer shell and a plurality of openings in the distal shell are aligned, and
      ∘ such that the plurality of elongated members extends through the plurality of aligned openings, and
   wherein the outer shell is displaceable relative to the distal shell.
26. Method according to item 25 comprising providing a fixation member configured to fixate the outer shell in an engaged or a disengaged position relative to the distal shell.
27. Method according to any of the items 25-26 comprising providing a second brace part substantially similar to the first brace part and attaching the first brace part and the second brace part.

### LIST OF REFERENCES

- 2: brace
- 10: first brace part
- 12: first primary brace part end
- 14: first secondary brace part end
- 15: distal side of distal shell
- 16: distal shell
- 18: proximal side of distal shell
- 19: distal side of compressible structure
- 20: compressible structure
- 21: proximal side of compressible structure
- 22: first height
- 24: first region
- 26: second region
- 28: opening(s) of distal shell
- 28': first opening of distal shell
- 30: elongated member
- 32: first elongated member
- 34: second elongated member
- 36: first primary point
- 38: first secondary point
- 40: second brace part
- 42: second primary brace part end
- 44: second secondary brace part end
- 46: second distal shell
- 47: distal side of second distal shell
- 48: proximal side of second distal shell
- 50: second compressible structure
- 52: second height
- 58: proximal layer
- 60: outer shell
- 62: proximal side of outer shell
- 64: distal side of outer shell
- 66: opening(s) of outer shell
- 66': first opening of outer shell
- 70: anatomical structure
- 72: primary anatomical structure
- 74: secondary anatomical structure
- 76: joint
- 78: injury
- 80: first part of anatomical structure
- 82: second part of anatomical structure
- 90: fixation member
- 92: locking mechanism

- h: height direction

## Claims

1. A brace for management of an anatomical structure comprising a primary anatomical structure, a secondary anatomical structure, and a joint and/or an injury between the primary anatomical structure and the secondary anatomical structure,
the brace comprising a first brace part extending from a first primary brace part end to a first secondary brace part end and being adapted to be positioned to cover a first part of the anatomical structure extending over the joint and/or injury such that the first primary brace part end is located on the primary anatomical structure and the first secondary brace part end is located on the secondary anatomical structure,
the first brace part comprises:
- a distal shell comprising a rigid material and having a proximal side and a distal side,
- a compressible structure having a proximal side and a distal side and comprising a first region, the compressible structure being elastically and non-permanently deformable in a height direction normal to the proximal side of the distal shell, and the compressible structure being arranged proximal to the distal shell,
- a plurality of elongated members including a first elongated member,
∘ wherein the first elongated member extends between a first primary point and a first secondary point in the height direction,
∘ wherein the first primary point is arranged at and travels along with the proximal side of the compressible structure within the first region of the compressible structure,
∘ wherein the first secondary point of the first elongated member is in a primary position relative to the distal shell when the first region of the compressible structure is in a non-compressed state, and wherein the first secondary point is in a secondary position relative to the distal shell when the first region of the compressible structure is in a compressed state,
wherein the first brace part comprises a locking mechanism, wherein the locking mechanism, in a locked state, prevents movement between the first secondary point of the first elongated member and the distal shell.

2. Brace according to claim 1, wherein the distal shell comprises a plurality of openings, including a first opening, and wherein the first elongated member is configured to extend through the first opening.

3. Brace according to any of the preceding claims, wherein the plurality of elongated members includes a second elongated member,
- wherein the second elongated member extends between a second primary point and a second secondary point in the height direction,
- wherein the second primary point is arranged at and travels along with the proximal side of the compressible structure within a second region of the compressible structure,
- wherein the second secondary point of the second elongated member is in a primary position relative to the distal shell when the second region of the compressible structure is in a non-compressed state, and wherein the second secondary point is in a secondary position relative to the distal shell when the second region of the compressible structure is in a compressed state,
wherein the locking mechanism, in the locked state, prevents movement between the second secondary point of the second elongated member and the distal shell.

4. Brace according to any of the preceding claims, wherein the compressible structure comprises at least one lattice structure.

5. Brace according to any of the preceding claims wherein the compressible structure is configured to be reversibly compressible between the non-compressed state and the compressed state a plurality of times, such as at least 10 times.

6. Brace according to any of the preceding claims as dependent on claim 2, wherein the locking mechanism comprises an outer shell comprising a proximal side and a distal side and a plurality of openings, wherein the outer shell is arranged such that the proximal side of the outer shell faces the distal side of the distal shell,
wherein the plurality of openings of the outer shell and the plurality of openings in the distal shell are aligned, and wherein the plurality of elongated members is configured to extend through the plurality of aligned openings, and
wherein the outer shell is displaceable relative to the distal shell from a disengaged position to an engaged position, such as to transition the locking mechanism to the locked state.

7. Brace according to claim 6 wherein the locking mechanism further comprises a fixation member configured to fixate the outer shell in the engaged or disengaged position.

8. Brace according to any of the claims 6-7 wherein the outer shell is displaceable relative to the distal shell in a direction substantially perpendicular to the height direction such as to modify the alignment of the openings.

9. Brace according to any of the preceding claims wherein the outer shell is biased towards the engaged or disengaged position.

10. Method for manufacturing a brace for management of an anatomical structure comprising a primary anatomical structure, a secondary anatomical structure, and a joint and/or an injury between the primary anatomical structure and the secondary anatomical structure, the method comprising:
- providing a distal shell for a first brace part, the distal shell comprising a rigid material and having a proximal side and a distal side,
- providing a compressible structure and a plurality of elongated members,
∘ the compressible structure having a proximal side and a distal side and comprising a first region, the compressible structure being elastically and non-permanently deformable,
∘ the plurality of elongated members includes a first elongated member, wherein the first elongated member extends between a first primary point and a first secondary point in the height direction,
- arranging the compressible structure and the plurality of elongated members,
∘ such that the compressible structure is arranged proximal to the distal shell, and the compressible structure being elastically and non-permanently deformable in a height direction normal to the proximal side of the distal shell,
∘ such that the first primary point of the first elongated member is arranged at, and travels along with, the proximal side of the compressible structure within the first region of the compressible structure,
∘ such that the first secondary point of the first elongated member is in a primary position relative to the distal shell when the first region of the compressible structure is in a non-compressed state, and
∘ such that the first secondary point of the first elongated member is in a secondary position relative to the distal shell when first region of the compressible structure is in a compressed state,
- providing a locking mechanism for the first brace part, wherein the locking mechanism, in a locked state, prevents movement between the first secondary point of the first elongated member and the distal shell.

11. Method according to claim 10, wherein providing the compressible structure comprises manufacturing the compressible structure using additive manufacturing, such as 3D printing.

12. Method according to any of the claims 10-11, wherein providing the compressible structure and the plurality of elongated members comprises manufacturing the compressible structure and the plurality of elongated members in a simultaneous additive manufacturing process, such as a 3D printing process.

13. Method according to any of the claims 10-12 comprising:
- providing an outer shell comprising a proximal side and a distal side and a plurality of openings,
- arranging the outer shell
∘ such that the proximal side of the outer shell faces the distal side of the distal shell,
∘ such that the plurality of openings of the outer shell and a plurality of openings in the distal shell are aligned, and
∘ such that the plurality of elongated members extends through the plurality of aligned openings, and
wherein the outer shell is displaceable relative to the distal shell.

14. Method according to claim 13 comprising providing a fixation member configured to fixate the outer shell in an engaged or a disengaged position relative to the distal shell.

15. Method according to any of the claims 10-14 comprising providing a second brace part substantially similar to the first brace part and attaching the first brace part and the second brace part.
